(19) Europäisches Patentamt
European Patent Office
Office européen des brevets

(11) EP 3 106 155 A1

(12) **EUROPEAN PATENT APPLICATION**

(43) Date of publication:
**21.12.2016 Bulletin 2016/51**

(21) Application number: **15305921.7**

(22) Date of filing: **15.06.2015**

(51) Int Cl.:
***A61K 31/00*** *(2006.01)* ***G01N 33/50*** *(2006.01)*
***A61P 7/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicants:
- **Universite d'Aix Marseille**
  **13284 Marseille Cedex 07 (FR)**
- **Assistance Publique Hôpitaux de Marseille**
  **13354 Marseille Cedex 5 (FR)**
- **INSTITUT NATIONAL DE LA SANTE ET DE LA RECHERCHE MEDICALE (INSERM)**
  **75013 Paris (FR)**

(72) Inventors:
- **BADENS, Catherine**
  **13006 Marseille (FR)**
- **THURET, Isabelle**
  **13190 Allauch (FR)**
- **GUIZOUARN, Hélène**
  **06000 Nice (FR)**

(74) Representative: **Gevers & Orès**
**41 avenue de Friedland**
**75008 Paris (FR)**

# (54) TREATMENT AND DIAGNOSIS OF HEREDITARY XEROCYTOSIS

(57) The invention relates to an *in vitro* method of diagnosis of hereditary xerocytosis in a subject, comprising genotyping the *KCNN4* gene encoding the Gardos channel in said subject. The invention also relates to an inhibitor of the KCNN4 protein for use in the treatment of hereditary xerocytosis, in particular in a human subject who is a carrier of the missense mutation c.1055G>A in the *KCNN4* gene.

EP 3 106 155 A1

SEQUENCE LISTING

<110> UNIVERSITE D'AIX MARSEILLE
ASSISTANCE PUBLIQUE-HOPITAUX DE MARSEILLE
INSTITUT NATIONAL DE LA SANTE ET DE LA RECHERCHE MEDICALE

<120> TREATMENT AND DIAGNOSIS OF HEREDITARY XEROCYTOSIS

<130> F2245/17-XRN/clg

<160> 14

<170> PatentIn version 3.5

<210> 1
<211> 2240
<212> DNA
<213> Homo sapiens

<220>
<221> CDS
<222> (397)..(1680)

<400> 1

```
gtccttcggt gtctgggtgt ggtgagtaga ggtgtgtgtc acaaagtaca gaccattgtg      60

tgtgacaaag cccatcgtgt gtctgtgtgt gtctttatcc acgtggatgg acgtctcttt     120

cttgctctgc cccaagacac accctagccc ctccttattc tcaaaagggg gagctgggga     180

gcctccccct accctggggc ctcccctgcc cctccccgcc ctgcctggcc gtcaccactc     240

cccagagggc acagggctct gctgtgcctc agagcaaaag tcccagagcc agcagagcag     300

gctgacgacc tgcaagccac agtggctgcc ctgtgcgtgc tgcgaggtgg gggaccctgg     360

gcaggaagct ggctgagccc caagaccccg gggcc atg ggc ggg gat ctg gtg        414
                                       Met Gly Gly Asp Leu Val
                                       1               5

ctt ggc ctg ggg gcc ttg aga cgc cga aag cgc ttg ctg gag cag gag      462
Leu Gly Leu Gly Ala Leu Arg Arg Arg Lys Arg Leu Leu Glu Gln Glu
            10                  15                  20

aag tct ctg gcc ggc tgg gca ctg gtg ctg gca gga act ggc att gga      510
Lys Ser Leu Ala Gly Trp Ala Leu Val Leu Ala Gly Thr Gly Ile Gly
        25                  30                  35

ctc atg gtg ctg cat gca gag atg ctg tgg ttc ggg ggg tgc tcg tgg      558
Leu Met Val Leu His Ala Glu Met Leu Trp Phe Gly Gly Cys Ser Trp
    40                  45                  50

gcg ctc tac ctg ttc ctg gtt aaa tgc acg atc agc att tcc acc ttc      606
Ala Leu Tyr Leu Phe Leu Val Lys Cys Thr Ile Ser Ile Ser Thr Phe
55                  60                  65                  70

tta ctc ctc tgc ctc atc gtg gcc ttt cat gcc aaa gag gtc cag ctg      654
Leu Leu Leu Cys Leu Ile Val Ala Phe His Ala Lys Glu Val Gln Leu
                75                  80                  85

ttc atg acc gac aac ggg ctg cgg gac tgg cgc gtg gcg ctg acc ggg      702
Phe Met Thr Asp Asn Gly Leu Arg Asp Trp Arg Val Ala Leu Thr Gly
```

```
            90                  95                  100

cgg cag gcg gcg cag atc gtg ctg gag ctg gtg gtg tgt ggg ctg cac          750
Arg Gln Ala Ala Gln Ile Val Leu Glu Leu Val Val Cys Gly Leu His
        105                 110                 115

ccg gcg ccc gtg cgg ggc ccg ccg tgc gtg cag gat tta ggg gcg ccg          798
Pro Ala Pro Val Arg Gly Pro Pro Cys Val Gln Asp Leu Gly Ala Pro
    120                 125                 130

ctg acc tcc ccg cag ccc tgg ccg gga ttc ctg ggc caa ggg gaa gcg          846
Leu Thr Ser Pro Gln Pro Trp Pro Gly Phe Leu Gly Gln Gly Glu Ala
135                 140                 145                 150

ctg ctg tcc ctg gcc atg ctg ctg cgt ctc tac ctg gtg ccc cgc gcc          894
Leu Leu Ser Leu Ala Met Leu Leu Arg Leu Tyr Leu Val Pro Arg Ala
                155                 160                 165

gtg ctc ctg cgc agc ggc gtc ctg ctc aac gct tcc tac cgc agc atc          942
Val Leu Leu Arg Ser Gly Val Leu Leu Asn Ala Ser Tyr Arg Ser Ile
            170                 175                 180

ggc gct ctc aat caa gtc cgc ttc cgc cac tgg ttc gtg gcc aag ctt          990
Gly Ala Leu Asn Gln Val Arg Phe Arg His Trp Phe Val Ala Lys Leu
        185                 190                 195

tac atg aac acg cac cct ggc cgc ctg ctg ctc ggc ctc acg ctt ggc         1038
Tyr Met Asn Thr His Pro Gly Arg Leu Leu Leu Gly Leu Thr Leu Gly
    200                 205                 210

ctc tgg ctg acc acc gcc tgg gtg ctg tcc gtg gcc gag agg cag gct         1086
Leu Trp Leu Thr Thr Ala Trp Val Leu Ser Val Ala Glu Arg Gln Ala
215                 220                 225                 230

gtt aat gcc act ggg cac ctt tca gac aca ctt tgg ctg atc ccc atc         1134
Val Asn Ala Thr Gly His Leu Ser Asp Thr Leu Trp Leu Ile Pro Ile
                235                 240                 245

aca ttc ctg acc atc ggc tat ggt gac gtg gtg ccg ggc acc atg tgg         1182
Thr Phe Leu Thr Ile Gly Tyr Gly Asp Val Val Pro Gly Thr Met Trp
            250                 255                 260

ggc aag atc gtc tgc ctg tgc act gga gtc atg ggt gtc tgc tgc aca         1230
Gly Lys Ile Val Cys Leu Cys Thr Gly Val Met Gly Val Cys Cys Thr
        265                 270                 275

gcc ctg ctg gtg gcc gtg gtg gcc cgg aag ctg gag ttt aac aag gca         1278
Ala Leu Leu Val Ala Val Val Ala Arg Lys Leu Glu Phe Asn Lys Ala
    280                 285                 290

gag aag cac gtg cac aac ttc atg atg gat atc cag tat acc aaa gag         1326
Glu Lys His Val His Asn Phe Met Met Asp Ile Gln Tyr Thr Lys Glu
295                 300                 305                 310

atg aag gag tcc gct gcc cga gtg cta caa gaa gcc tgg atg ttc tac         1374
Met Lys Glu Ser Ala Ala Arg Val Leu Gln Glu Ala Trp Met Phe Tyr
                315                 320                 325

aaa cat act cgc agg aag gag tct cat gct gcc cgc agg cat cag cgc         1422
Lys His Thr Arg Arg Lys Glu Ser His Ala Ala Arg Arg His Gln Arg
            330                 335                 340

aag ctg ctg gcc gcc atc aac gcg ttc cgc cag gtg cgg ctg aaa cac         1470
```

```
Lys Leu Leu Ala Ala Ile Asn Ala Phe Arg Gln Val Arg Leu Lys His
        345                 350                 355

cgg aag ctc cgg gaa caa gtg aac tcc atg gtg gac atc tcc aag atg      1518
Arg Lys Leu Arg Glu Gln Val Asn Ser Met Val Asp Ile Ser Lys Met
    360                 365                 370

cac atg atc ctg tat gac ctg cag cag aat ctg agc agc tca cac cgg      1566
His Met Ile Leu Tyr Asp Leu Gln Gln Asn Leu Ser Ser Ser His Arg
375                 380                 385                 390

gcc ctg gag aaa cag att gac acg ctg gcg ggg aag ctg gat gcc ctg      1614
Ala Leu Glu Lys Gln Ile Asp Thr Leu Ala Gly Lys Leu Asp Ala Leu
                395                 400                 405

act gag ctg ctt agc act gcc ctg ggg ccg agg cag ctt cca gaa ccc      1662
Thr Glu Leu Leu Ser Thr Ala Leu Gly Pro Arg Gln Leu Pro Glu Pro
            410                 415                 420

agc cag cag tcc aag tag ctggacccac gaggaggaac caggctactt             1710
Ser Gln Gln Ser Lys
        425

tccccagtac tgaggtggtg gacatcgtct ctgccactcc tgacccagcc ctgaacaaag    1770

cacctcaagt gcaaggacca aagggggccc tggcttggag tgggttggct tgctgatggc    1830

tgctggaggg gacgctggct aaagtgggta ggccttggcc cacctgaggc cccaggtggg    1890

aacatggtca cccccactct gcataccctc atcaaaaaca ctctcactat gctgctatgg    1950

acgacctcca gctctcagtt acaagtgcag gcgactggag gcaggactcc tgggtccctg    2010

ggaaagaggg tactaggggc ccggatccag gattctggga ggcttcagtt accgctggcc    2070

gagctgaaga actgggtatg aggctggggc ggggctggag gtggcgcccc ctggtgggac    2130

aacaaagagg acaccatttt tccagagctg cagagagcac ctggtgggga ggaagaagtg    2190

taactcacca gcctctgctc ttatctttgt aataaatgtt aaagccagaa               2240


<210>  2
<211>  427
<212>  PRT
<213>  Homo sapiens

<400>  2

Met Gly Gly Asp Leu Val Leu Gly Leu Gly Ala Leu Arg Arg Arg Lys
1               5                   10                  15


Arg Leu Leu Glu Gln Glu Lys Ser Leu Ala Gly Trp Ala Leu Val Leu
            20                  25                  30


Ala Gly Thr Gly Ile Gly Leu Met Val Leu His Ala Glu Met Leu Trp
        35                  40                  45


Phe Gly Gly Cys Ser Trp Ala Leu Tyr Leu Phe Leu Val Lys Cys Thr
    50                  55                  60
```

```
Ile Ser Ile Ser Thr Phe Leu Leu Leu Cys Leu Ile Val Ala Phe His
65                  70                  75                  80

Ala Lys Glu Val Gln Leu Phe Met Thr Asp Asn Gly Leu Arg Asp Trp
                85                  90                  95

Arg Val Ala Leu Thr Gly Arg Gln Ala Ala Gln Ile Val Leu Glu Leu
            100                 105                 110

Val Val Cys Gly Leu His Pro Ala Pro Val Arg Gly Pro Pro Cys Val
        115                 120                 125

Gln Asp Leu Gly Ala Pro Leu Thr Ser Pro Gln Pro Trp Pro Gly Phe
    130                 135                 140

Leu Gly Gln Gly Glu Ala Leu Leu Ser Leu Ala Met Leu Leu Arg Leu
145                 150                 155                 160

Tyr Leu Val Pro Arg Ala Val Leu Leu Arg Ser Gly Val Leu Leu Asn
                165                 170                 175

Ala Ser Tyr Arg Ser Ile Gly Ala Leu Asn Gln Val Arg Phe Arg His
            180                 185                 190

Trp Phe Val Ala Lys Leu Tyr Met Asn Thr His Pro Gly Arg Leu Leu
        195                 200                 205

Leu Gly Leu Thr Leu Gly Leu Trp Leu Thr Thr Ala Trp Val Leu Ser
    210                 215                 220

Val Ala Glu Arg Gln Ala Val Asn Ala Thr Gly His Leu Ser Asp Thr
225                 230                 235                 240

Leu Trp Leu Ile Pro Ile Thr Phe Leu Thr Ile Gly Tyr Gly Asp Val
                245                 250                 255

Val Pro Gly Thr Met Trp Gly Lys Ile Val Cys Leu Cys Thr Gly Val
            260                 265                 270

Met Gly Val Cys Cys Thr Ala Leu Leu Val Ala Val Val Ala Arg Lys
        275                 280                 285

Leu Glu Phe Asn Lys Ala Glu Lys His Val His Asn Phe Met Met Asp
    290                 295                 300

Ile Gln Tyr Thr Lys Glu Met Lys Glu Ser Ala Ala Arg Val Leu Gln
```

305 310 315 320

Glu Ala Trp Met Phe Tyr Lys His Thr Arg Arg Lys Glu Ser His Ala
325 330 335

Ala Arg Arg His Gln Arg Lys Leu Leu Ala Ala Ile Asn Ala Phe Arg
340 345 350

Gln Val Arg Leu Lys His Arg Lys Leu Arg Glu Gln Val Asn Ser Met
355 360 365

Val Asp Ile Ser Lys Met His Met Ile Leu Tyr Asp Leu Gln Gln Asn
370 375 380

Leu Ser Ser Ser His Arg Ala Leu Glu Lys Gln Ile Asp Thr Leu Ala
385 390 395 400

Gly Lys Leu Asp Ala Leu Thr Glu Leu Leu Ser Thr Ala Leu Gly Pro
405 410 415

Arg Gln Leu Pro Glu Pro Ser Gln Gln Ser Lys
420 425

<210> 3
<211> 25
<212> DNA
<213> Artificial Sequence

<220>
<223> Probe

<400> 3
cccacaggtt ccgccaggtg cggct 25

<210> 4
<211> 25
<212> DNA
<213> Artificial Sequence

<220>
<223> Probe

<400> 4
cccacaggtt ccaccaggtg cggct 25

<210> 5
<211> 20
<212> DNA
<213> Artificial Sequence

<220>
<223> Primer forward

<400> 5
agtgctacaa gaagcctgga 20

<210> 6
<211> 20
<212> DNA
<213> Artificial Sequence

<220>
<223> Primer reverse

<400> 6
tgctaagcag ctcagtcagg 20

<210> 7
<211> 22
<212> PRT
<213> Homo sapiens

<400> 7

```
Lys Leu Leu Ala Ala Ile Asn Ala Phe Arg Gln Val Arg Leu Lys His
1               5                   10                  15

Arg Lys Leu Arg Glu Gln
            20
```

<210> 8
<211> 21
<212> PRT
<213> Pan troglodytes

<400> 8

```
Lys Leu Leu Ala Ala Ile Asn Ala Phe Arg Gln Val Arg Leu Lys His
1               5                   10                  15

Arg Lys Leu Arg Glu
            20
```

<210> 9
<211> 16
<212> PRT
<213> Macaca mulatta

<400> 9

```
Ile Asn Ala Phe Arg Gln Val Arg Leu Lys His Arg Lys Leu Gln Glu
1               5                   10                  15
```

<210> 10
<211> 18
<212> PRT
<213> Felis catus

<400> 10

```
Arg Leu Leu Ala Ala Ile Asn Arg Phe Arg Gln Val Arg Leu Lys His
1               5                   10                  15


Arg Lys



<210>  11
<211>  18
<212>  PRT
<213>  Mus musculus

<400>  11

Lys Met Leu Ala Ala Ile His Thr Phe Arg Gln Val Arg Leu Lys His
1               5                   10                  15


Arg Lys



<210>  12
<211>  21
<212>  PRT
<213>  Drosophila melanogaster

<400>  12

Lys Phe Leu Leu Ala Ile Tyr Ala Leu Arg Lys Val Lys Met Asp Gln
1               5                   10                  15


Arg Lys Leu Met Asp
            20


<210>  13
<211>  16
<212>  PRT
<213>  Caenorhabditis elegans

<400>  13

Lys Phe Leu Leu Ala Ile Tyr Glu Met Arg Arg Val Arg Arg Asp Gln
1               5                   10                  15


<210>  14
<211>  21
<212>  PRT
<213>  Xenopus tropicalis

<400>  14

Asn Leu Leu Arg Ala Ile His Val Phe Arg Arg Ser Arg Ile Ser His
1               5                   10                  15


Lys Asn Leu Lys Asp
            20
```

## Description

The present invention relates to the diagnosis of hereditary xerocytosis and the treatment of this disorder.

Water and solute homeostasis is essential for the maintenance of erythrocyte integrity and is controlled via the regulation of monovalent cation content. Several primary disorders of erythrocytes hydration exist and are characterized by an abnormal permeability of the erythrocyte membrane to sodium and potassium, resulting either in swelling or shrinkage of red cells (Rinehart *et al.*, 2010). Clinically, these inherited disorders are associated with chronic hemolytic anemia and are due to defects in various transmembrane ion channels or transporters (Da Costa L, *et al.*, 2013).

Hereditary Xerocytosis, (HX) ([OMIM] 194380), is an autosomal dominant congenital hemolytic anemia characterized by primary erythrocyte dehydration (Miller *et al.*, 1971). In HX patients, red blood cells exhibit an altered intracellular cation content and cellular dehydration which is responsible for an increased erythrocyte mean corpuscular hemoglobin concentration (MCHC) and decreased erythrocyte osmotic fragility (Archer *et al.*, 2014). Under the microscope, blood films show various shape abnormalities, the most characteristic being a central pallor, straight or crescent-shaped, which leads to the denomination of stomatocyte for these cells and of Dehydrated Hereditary Stomatocytosis as an alternative name for HX (Da Costa *et al.*, 2013).

HX has been associated with missense mutations in FAM38A encoding the red cell membrane mechanosensitive cation channel, PIEZO1 (Zarychanski *et al.*, 2012; Andolfo *et al.*, 2013a). Functional studies have demonstrated that in PIEZO1, the mutations slowed channel inactivation and introduced a pronounced latency for activation (Bae *et al.*, 2013). More recently, another type of red cell ion exchange defect associated with pseudohyperkalemia has been linked to mutations in the ATP binding cassette transporter ABCB6 (Andolfo *et al.*, 2013b). Rinehart *et al.* (2010) report that a locus for hereditary xerocytosis has been mapped to 16q23-q24, but the affected gene has not yet been identified.

The Gardos channel is a cation channel also referred to as KCa3.1 or KCNN4. It is a $Ca^{2+}$ sensitive, intermediate conductance, potassium selective channel, initially described in pancreas cells but present in many cell types including erythrocytes (Maher and Kuchel, 2003). The locus of the gene encoding the Gardos channel (KCNN4 protein) is mapped 19q13.2. The Gardos channel is made of 4 identical subunits; each subunit is encoded by a single gene, *KCNN4,* and comprises 6 transmembrane domains and a pore region between the $5^{th}$ and the $6^{th}$ transmembrane domains (Figure 2D) (Maher and Kuchel, 2003). In steady state conditions, the Gardos channel is inactive. Its function is not fully elucidated in mature normal erythrocytes. Under external stimulation, intracellular $Ca^{2+}$ increases and then interacts with Calmodulin molecules that are bound tightly on each of the four channel subunits of the Gardos channel. $Ca^{2+}$ binding to Calmodulin results in the opening of the channel and rapid $K^+$ and water efflux leading to erythrocyte dehydration and shrinkage, a mechanism referred to as the Gardos effect (Maher and Kuchel, 2003; Fanger *et al.*, 1999). Red blood cells are in constant movement during blood circulation where they experience mechanical stress on their membrane. Using on-cell patch clamp experiments, it has been shown that local membrane deformation can act as a stimulating event in red cells and lead to Gardos activation, suggesting that this mechanosensory mechanism may allow erythrocytes to adapt their volume and shape to pass through the narrow capillaries of the microvasculature (Dyrda *et al.*, 2010). A number of recent studies have described its role in a variety of physiological events and pointed it out as an interesting therapeutic target in a large panel of human diseases (Wulff and Köhler, 2013; Wulff and Castle, 2010).

The inventors have identified a missense mutation (p.Arg352His mutation) located in one of the functional regions of the Gardos channel and its association with chronic hemolysis and dehydrated cells in two unrelated HX families with eight affected HX persons. The affected individuals present chronic anemia that varies in severity. Their red cells exhibit a panel of various shape abnormalities such as elliptocytes, hemighosts, schizocytes and very rare stomatocytic cells. The missense mutation concerns a highly conserved residue among species, located in the region interacting with Calmodulin and responsible for the channel opening and the $K^+$ efflux.

The diagnostic of this disorders in these persons was prevented by the fact that two of the tests which could have led to biological diagnosis are no longer performed in routine laboratories: Osmotic Resistance has very often been replaced by the EMA test, which is normal in the present cases, and intra-erythrocytic $K^+$ determination is currently no longer offered on a routine basis.

The provision of a new diagnostic test of hereditary xerocytosis is therefore of clinical interest.

The functional experiments performed on *Xenopus oocytes* showed that the channel mutated on residue 352 is normally activated by $Ca^{2+}$ influx, permits an efflux of $K^+$ of increased intensity when compared to the wild-type (wt) channel and remains open and active during a prolonged period when compared to the normal channel. It is likely that the mutation, removing a positive charge in the Calmodulin binding domain of the Gardos channel, modifies interactions with this activating partner, resulting in a more active channel. Experiments on the human cell line HEK293 confirmed the higher current density for mutated KCNN4. Despite the mutation in the Calmodulin binding site, the trafficking properties of the mutant are similar and unaffected in cells with very different trafficking properties (HEK293 and *Xenopus oocytes*). These experiments showed that p.Arg352His mutation changes $Ca^{2+}$ sensitivity of the channel that is activated by 10 times lower $Ca^{2+}$ concentration. The anomaly in the kinetic of activation combined with a higher sensitivity to $Ca^{2+}$ confers pathogenicity to p.Arg352His KCNN4.

The description of such a molecular defect provides a rationale for the treatment of this type of HX by specific inhibitors of the Gardos channel such as the Senicapoc, which was tested in the past in a phase III study for the treatment of Sickle Cell disease and was proven, on this occasion, to be non-toxic (Ataga *et al.*, 2011).

Accordingly, the present invention provides an inhibitor of the Gardos channel (KCNN4 protein) for use in the treatment of hereditary xerocytosis.

The Gardos channel is a $Ca^{2+}$ sensitive, intermediate conductance, potassium selective channel also referred to as KCa3.1 or KCNN4 (Maher and Kuchel, 2003). The Gardos channel can be a wild-type Gardos channel or a mutant Gardos channel, such as the Gardos channel (KCNN4 protein) variant p.Arg352His.

In a preferred embodiment, the Gardos channel is from human origin. The amino acid sequence of the wild-type human Gardos channel (KCNN4 protein) is available under accession number 015554 (GI:17366160) in the Uni-ProtKB database, and referred herein to as SEQ ID NO: 2.

An inhibitor of the Gardos channel refers to a selective Gardos channel blocker that specifically inhibits the efflux of potassium from the erythrocytes.

An inhibitor of the Gardos channel can be identified by screening a collection of candidate compounds for their ability to specifically inhibit the efflux of potassium from the erythrocytes. Methods for measuring the inhibition of the efflux of potassium from the erythrocytes are known in themselves. Examples of such methods are described in Brugnara *et al.*, 1993a and 1993b; Ellory *et al.*, 1994. Both the percent inhibition of the Gardos channel and the $IC_{50}$ of an inhibitor of the Gardos channel can be assayed utilizing the methods described in Brugnara *et al.*, 1993b.

The potency of an inhibitor of the Gardos channel can be assayed using erythrocytes by a method such as that disclosed by Brugnara *et al.*, 1993a.

Inhibitors of the Gardos channel include organic molecules, amino acids and antibodies.

The antibodies can be polyclonal or monoclonal antobodies. The term "antibody" or "antibodies" as used herein also encompasses functional fragments of antibodies, including fragments of chimeric, humanized, single chain antibodies or fragments thereof (*e.g.*, Fv, Fab, Fab'and F(ab') 2 fragments). Suitable antibodies are those which are directed to KCNN4 protein (Gardos channel). Advantageously, said antibody is a monoclonal antibody, or fragment thereof.

In a preferred embodiment, the inhibitor of the Gardos channel is selected from the group consisting of imidazole antimycotics (Brugnara *et al.*, 1996), such as clotrimazole (Brugnara *et al.*, 1993a) metronidazole (Brugnara *et al.*, 1993a), econazole (Brugnara *et al.*, 1993a); arginine (Romero *et al.*, 2002); Tram-34 (1-[(2-Chlorophenyl)diphenylmethyl]-1H-pyrazole) (Wulff *et al.*, 2000); Charybdotoxin; Maurotoxin (Castle *et al.*, 2002); nifedipine (Brugnara *et al.*, 1993a); Nitrendipine (Brugnara *et al.*, 1993a); inhibitors of calcium activated potassium flux that display selectivity and a potency towards the Gardos channel described in International Applications WO 00/50026, WO 2004/016221, WO 2005/113490 and WO 2006/084031, including senicapoc (ICA-17043; bis(4-fluorophenyl)phenyl acetamide; Ataga *et al.*, 2008; 2009), 2,2-Bis(4-fluorophenyl)-N-methoxy-2-phenylacetamidine, 2-(2-Chlorophenyl)-2,2-diphenylacetaldehyde oxime, 2-(2-Chlorophenyl)-2,2-bis(4-fluorophenyl)-N-hydroxyacetamidine, 2,2,2-Tris(4-fluorophenyl)-N-hydroxyacetamidine, 2-(2-Fluorophenyl)-2-(4-fluorophenyl)-N-hydroxy-2-phenylacetamidine, phosphoric acid 3-(2-oxazolyl)-4-[3-(trifluoromethyl)phenylsulfonamido]phenyl monoester, N-[2-(4,5-Dihydrooxazol-2-yl)phenyl]-3-(trifluoromethyl)benzenesulfonamide, N-[4-Methoxy-2-(2-oxazolyl)phenyl]benzenesulfonamide, N-[4,5-Dimethoxy-2-(3-methyl-1,2,4-oxadiazol-5-yl)phenyl]-3-(trifluoromethyl)benzenesulfonamide, N-[2-(2-Furyl)phenyl]-3-(trifluoromethyl)benzenesulfonamide and N-[4-Methyl-2-(2-oxazolyl)phenyl]-3-(trifluoromethyl)benzenesulfonamide, preferably senicapoc (see also Stocker *et al.*, 2003).

Said inhibitor of the Gardos channel can be administered by itself, or mixed with suitable carriers or excipient(s). It can be used systemically. One can use any formulation suitable for systemic administration.

As used herein, the terms "treatment" or "treating" includes the administration of an inhibitor of the Gardos channel as defined above to a subject who has hereditary xerocytosis, with the purpose to alleviate, relieve, alter, remedy, ameliorate, improve or affect this disorder.

The subject is preferably a human subject, more preferably a human subject who is a carrier for the missense mutation c.1055G>A in the *KCNN4* gene encoding the Gardos channel, resulting in an amino acid change from arginine to histidine in codon 352 (p.Arg352His).

The nucleic acid sequence of the wild-type human *KCNN4* gene encoding the Gardos channel (Map:19q13.2) is available under the accession number NC_000019.10 (GI:568815579 ) in the NCBI GenBank database.

The nucleic acid sequence of the mRNA (cDNA) encoded by the wild-type human *KCNN4* gene is available under the accession number NM_002250.2 (GI:25777651) in the NCBI GenBank database, referred herein to as SEQ ID NO: 1.

The amino acid sequence of the wild-type human Gardos channel (KCNN4 protein) is available under accession number 015554 (GI:17366160) in the UniProtKB database or NM_002250.2 (GI:25777651) in the NCBI GenBank database, referred herein to as SEQ ID NO: 2.

Methods for identifying said mutation are described below.

The present invention also provides a method for treating hereditary xerocytosis, comprising administering to a subject in need thereof an inhibitor of the Gardos channel (KCNN4 protein) as defined above.

The present invention also provides the use of an inhibitor of the Gardos channel (KCNN4 protein) as defined above for the preparation of a medicament for treating hereditary xerocytosis.

The present invention also provides a method for genotyping, *in vitro,* the *KCNN4* gene in a human subject comprising the steps of:

(a) isolating mRNA or genomic DNA from a nucleic acid sample obtained from said subject,
(b) determining the nucleotide present at position c.1055 of the *KCNN4* gene encoding the Gardos channel.

As used herein, the term "determining the nucleotide corresponding to the nucleotide present at position c.1055 of the *KCNN4* gene encoding the Gardos channel" refers to determining the nucleotide corresponding to the nucleotide present at position c.1055 of the *KCNN4* gene encoding the Gardos channel, either in said isolated mRNA or genomic DNA.

Said subject is suffering or not from hereditary xerocytosis.

Methods for obtaining a nucleic acid sample from a subject are well known in the art. Methods for isolating mRNA or genomic DNA from a subject are also well known in the art.

Advantageously, the mRNA or genomic DNA can be obtained from a blood sample from said subject, in particular from reticulocytes from said subject for isolating mRNA or from white blood cells from said subject for isolating genomic DNA.

Methods for determining said nucleotide in step (b) comprise the methods for detecting a single nucleotide polymorphism (SNP) which are well known in this art.

Methods for detecting SNPs have been described in the prior art, including selective hybridization techniques (*e.g.*, reverse dot blot, Southern blot for DNAs, Northern blot for RNAs,), selective amplification, nucleic acid sequencing, restriction fragment length polymorphism (RFLP), amplified fragment length polymorphism (AFLP), ligation chain reaction (LCR), mass spectrometry (see for review Kaplan and Delpech, 2007).

In particular, to determine a SNP in mRNA or genomic DNA, it may be necessary to amplify the corresponding mRNA or genomic region respectively. For this, it can be used PCR primers whose nucleotide sequences may be obtained from the sequences containing the SNP to be amplified. The PCR amplified fragments can then be analyzed by sequencing (e.g., Sanger sequencing) or hybridization techniques. The PCR amplified fragments can also be analyzed on mass spectrometer through specific extension primers distinguishing the two variants (wild-type or variant) known at the polymorphic site.

By way of examples, a set of PCR primers as defined above include the set of PCR primers of SEQ ID NO: 5 and SEQ ID NO: 6.

A SNP in mRNA or genomic DNA can also be determined by reverse dot blot. One can use the probe of SEQ ID NO: 3 to determine the wild-type sequence and the probe of SEQ ID NO: 4 to determine the variant (mutant) sequence.

According to the method for genotyping it can be deduced that the subject is suffering from hereditary xerocytosis if the nucleotide present at position c.1055 of the *KCNN4* gene encoding the Gardos channel is adenine (A).

The present invention also provides an *in vitro* method of diagnosing the presence of or predisposition to hereditary xerocytosis in a human subject, comprising the step of:

(i) providing a biological sample from said subject and
(ii) detecting the presence of the missense mutation c.1055G>A in the *KCNN4* gene encoding the Gardos channel encoded by the *KCNN4* gene or the missense mutation p.Arg352His in the Gardos channel (KCNN4 protein), preferably detecting the presence of the missense mutation c.1055G>A in the *KCNN4* gene encoding the Gardos channel,

the presence of said mutation constituting a marker of a hereditary xerocytosis or a predisposition to hereditary xerocytosis in said subject.

As used herein the term "detecting the presence of the missense mutation c.1055G>A in the *KCNN4* gene" refers to detecting the presence of the mutation corresponding to missense mutation c.1055G>A in the *KCNN4* gene, either in mRNA or genomic DNA from a nucleic acid sample obtained from said subject.

The presence of the missense mutation c.1055G>A in the *KCNN4* gene encoding the Gardos channel can be detected by genotyping the *KCNN4* gene in said human subject as described above.

Methods for determining a point mutation in a protein are well known in this art (see for review Kaplan and Delpech, 2007). The presence of the p.Arg352His mutation in the Gardos channel (KCNN4 protein) can be detected by protein sequencing or binding to a ligand (such as an antibody) specifically directed to the Gardos channel (KCNN4 protein) variant p.Arg352His, in particular by western blot using antibodies specifically directed to the Gardos channel (KCNN4 protein) variant p.Arg352His, preferably by protein sequencing.

The present invention also provides a kit for diagnosing a hereditary xerocytosis comprising the probe of SEQ ID NO: 3 and/or the probe of SEQ ID NO: 4.

The present invention also provides the use of the probe of SEQ ID NO: 3 and/or the probe of SEQ ID NO: 4 for *in vitro* diagnosing a hereditary xerocytosis in a human subject.

The present invention also relates to methods for screening inhibitors of the Gardos channel. Such inhibitors are useful as selective Gardos channel blocker that specifically inhibits the efflux of potassium from the erythrocytes, and therefore for treating hereditary xerocytosis.

The methods include binding assays and/or functional assays, and may be performed *in vitro,* in cell systems (yeast, bacteria, *Xenopus* oocyte) or in animals, involving the human Gardos channel (KCNN4 protein) variant p.Arg352His.

For cell systems, cells can be native, *i.e.,* cells that normally express the Gardos channel (KCNN4 protein) variant p.Arg352His polypeptide, as a biopsy or expanded in cell culture. Preferably, these native cells are derived from erythrocytes. Alternatively, cells are recombinant host cells, in particular *Xenopus laevis* oocytes, expressing the Gardos channel (KCNN4 protein) variant p.Arg352His.

The present invention therefore provides an *in vitro* method for screening a biologically active inhibitor of the human Gardos channel (KCNN4 protein) variant p.Arg352His, said method comprising contacting *in vitro* a test compound with the human Gardos channel (KCNN4 protein) variant p.Arg352His and determining the ability of said test compound to prevent ion conductance through the channel when compared to the wild-type human Gardos channel (KCNN4 protein) of SEQ ID NO: 2, wherein preventing ion conductance through the channel when compared to the wild-type human Gardos channel (KCNN4 protein) of SEQ ID NO: 2 provides an indication as to the ability of the compound to inhibit the human Gardos channel (KCNN4 protein) variant p.Arg352His.

In a preferred embodiment of said method, the method comprises expressing a plasmid containing the mutated cDNA encoding the human Gardos channel (KCNN4 protein) variant p.Arg352His in *Xenopus laevis* oocytes and measuring the current voltage in the presence of the test compound; a decrease in the conductance indicating that said test compound inhibits the human Gardos channel (KCNN4 protein) variant p.Arg352His.

In another preferred embodiment of said method, erythrocytes expressing the Gardos channel (KCNN4 protein) variant p.Arg352His polypeptide are exposed to a test compound and a Rb-containing medium. The initial rate of $^{86}$Rb transport can be calculated from a parameter such as the linear least square slope of $^{86}$Rb uptake by the erythrocytes. Inhibitory constants can be calculated by standard methods using computer-assisted nonlinear curve fitting.

A method for measuring current voltage in *Xenopus laevis* oocytes is described in the Example below.

In addition to the above features, the invention further comprises other features which will emerge from the following description, which refers to the identification of the p.Arg352His mutation in the Gardos channel and its association with chronic hemolysis and dehydrated cells, as well as to the appended figures:

**Figure 1:** Red blood cell and DNA investigations. **A:** Family pedigrees showing mutation segregation; **B:** Blood film smears (MGG) for the proband 1 and his mother; **C:** Multiple interspecies protein sequence alignment of KCNN4 in the region of residue 352; **D:** KCNN4 transcript sequencing: upper panel: wild type sequence; bottom panel: transcript with mutation c.1055G>A (p.Arg352His). **E:** Red cell Osmotic fragility test using osmolar gradient ranging from 0.1 to 1% of NaCl solution. **A:** at T0 and at 37°C for a Control, Mother and Proband from Family 1. **B:** after T24h incubation at 4° or 37°C for a control and the proband.

**Figure 2:** Functional analysis of the Gardos channel variant p.Arg352His. **A:** Activation kinetic. For oocytes expressing WT KCNN4 or p.Arg352His KCNN4, the current at 0 mV was plotted as a function of time (left panel). The maximal intensity of the current being different between WT and mutated KCNN4, a ratio between I at different times and the Imax was calculated for each condition and plotted as a function of time. Data are means of 15 (WT) or 22 (p.Arg352His) oocytes coming from 3 different batches. The arrow indicates the opening of calcium ionophore perfusion. The bar graph (right panel) quantifies the remaining current at 220 s in oocytes expressing WT or p.Arg352His KCNN4. The current at 220 s was divided by Imax (at about 135 s) for each recording. Data are means +/- sem of 15 (WT) or 22 (p.Arg352His) oocytes. Statistical analysis were done using the Mann and Whitney test, the two bars are different with a risk of 0.2% (bidirectional). **B:** Current-voltage curves of WT and mutated KCNN4 in oocyte membranes with quantification. I/V curves correspond to the maximal current recorded for WT or p.Arg352His KCNN4 expressing oocytes (around 135 s) in gluconate medium with 1 $\mu$M A23187. Data are means of ramps recorded on 15 (WT) or 22 (p.Arg352His) oocytes. NI are control (non-injected) oocytes (n=4). Inset: western blot detection of WT and mutated KCNN4 indicated by the arrow (around 50 kDa). **C:** TRAM-34 inhibition: once the maximal current was reached in oocytes expressing p.Arg352His mutant, 10 $\mu$M TRAM-34 was added. This induced a rapid current decrease. The mean value of maximal currents at 50 mV was calculated (white bar) and compared to the mean value of minimal currents at 50 mV after TRAM-34 addition (grey bar). Data are means of 4 oocytes +/-sem.

**Figure 3:** KCNN4 expression in HEK293 cells. **A:** Activation kinetic of wt and p.Arg352His KCNN4 recorded in whole cell configuration. WT KCNN4 or p.Arg352His KCNN4 were expressed in HEK293 cells and then subjected to patch-clamp experiment in whole cell configuration. Current were recorded immediately after break-in using a 150ms voltage ramp protocol from -120 to +80 mV from an holding potential of -60 mV. The current at -20 mV was plotted as a function of time. Values are mean ± SEM of 12-8 experiments. **B:** Representative current/voltage curves for HEK293 expressing wt or p.Arg352His KCNN4. Inset (upper left) represents reversal potentials just after break-in and at the steady state. Values are represented as a Tukey's plot (n=12-8) Statistical analyses were done using Kruskal and Wallis test followed by a Tukey post-hoc test. C: Tukey's plots showing current density at -20mV in wt and mutated sk4 (n=12-8; *** p< 0.001). Statistical analysis was performed using a Mann and Whitney test. **D:** Representative traces of $Ca^{2+}$-dependent activation of wt and p.Arg352His KCNN4 current recorded in an inside-out macropatch configuration. Currents were elicited by 150 ms voltage ramps from -120 to +80 mV. Each trace corresponds to a different concentration of $Ca^{2+}$ indicated on the right hand side of the I/V. **E:** Normalized $K^+$ current measured at -45 mV in response to $[Ca^{2+}]i$ was plotted as a function of $[Ca^{2+}]I$ for wt (squares) and p.Arg352His mutated KCNN4 (circles). The experimental values (mean ± SEM) were fitted with the Hill equation (Origin software (Northampton, MA)):

$$Y = \frac{Ymax\ [Ca^{2+}]_i^n}{(K_{0.5})^n + [Ca^{2+}]_i^n}$$

where Y is relative KCNN4 current at -45 mV (I/Imax) for each $[Ca^{2+}]$, Ymax is the maximum current (Imax), K0.5 is the apparent dissociation constant, and n is the Hill coefficient. Insert (lower right) show Hill equation parameter K0.5 and nh. Values are represented as Tukey's plot (n=4, *p<0. 05). Statistical analysis was performed using a Mann and Whitney test.

**Figure 4:** Cation contents and cell volume as a function of incubation time with vanadate. A: $K^+$ content, **B:** cell water and **C:** $Na^+$ contents in red cells incubated with 5 mM vanadate (black circles for control red cells, black squares for patient red cells) or 5mM vanadate with 10 μM TRAM-34 (grey circles for control red cells and grey squares for patient red cells). Data in μmol per g of dry weight, are mean ± S.D., n=3.

## EXAMPLE: IDENTIFICATION OF THE P.ARG352HIS MUTATION IN THE GARDOS CHANNEL ASSOCIATED WITH HEREDITARY XEROCYTOSIS

### 1. Material and methods

**Hematological tests:** An osmotic fragility test, based on the observation of the fragility of red blood cells in hypotonic saline solutions, was performed immediately after sampling and after 24 hours' incubation at 4° or 37°C.

**NMR:** NMR experiments were performed on a 400 AVANCE wide-bore spectrometer (Bruker Biospin, Billerica, MA), using stimulation by the ionophore A23187 (Sigma Aldrich).

**NGS Sequencing:** Exome sequencing was performed after exome enrichment using Ion AmpliSeq™ (Thermo Fisher Scientific Inc., Waltham, MA USA), template preparation using the Ion PI™ Template OT2 200 Kit v2 on the Ion OneTouch™ 2 System and sequencing using the Ion PI™ Chip Kit v2 and Ion PI™ Sequencing 200 Kit v2 on the Ion Proton™ Sequencer (Thermo Fisher Scientific Inc., Waltham, MA USA). Raw data were first aligned with the provided software suite to generate BAM files. The coverage and sequencing depth analysis were computed using the BEDtools suite v2.17 (Quinlan and Hall, 2010) and in-house scripts. Variants were identified using the Torrent Browser Variant caller (version 4.0.2), annotated and prioritized with the in-house "VarAFT" system that includes Annovar (Wang *et al.*, 2010).

The mutation was confirmed on DNA samples and KCNN4 transcripts from fresh reticulocytes by Sanger sequencing (3500XL Genetic AnalyzerR, Life Technologies, Carlsbad, CA).

**Expression in *Xenopus* oocytes:** Plasmid pcDNA3KCNN4-HA (Joiner *et al.*, 2001) was used to introduce the point mutation p.Arg352His by PCR. A Hemagglutinin tag (HA) was present in the C-terminal end of KCNN4 (Joiner *et al.*, 1997). Female *Xenopus laevis* were anaesthetized with MS222 according to the procedure recommended by ethics committee of the applicants. Oocytes were harvested and injected as previously published (Barneaud-Rocca *et al.*, 2011).

Current recording was performed as follow: a ramp protocol between -120 to +80 mV for 2 seconds, holding potential -80 mV, was applied using Clampex (PClamp, Molecular Devices Corporation). To avoid looking at chloride channel activation, current recording was done in MBS where chloride was substituted by gluconate (Na-gluconate 85 mM and K-gluconate 1 mM). Junction potential was minimized using an agar bridge and KCl 3M. Electrodes filled with KCl 3M were 0.5 MOhm resistance. After equilibration in this gluconate MBS, KCNN4 was activated by the calcium ionophore A23187, 1 μM in MBS gluconate. In control oocytes, no current was activated by ionophore addition.

**Western blotting on oocyte:** Oocyte membrane were prepared as previously described (Martial *et al.*, 2007). Immunodetection of KCNN4-HA was done using an anti-HA antibody (1/1000, Sigma). To compare KCNN4 expression levels in different samples, the cell membrane marker β1 Na,KATPase was used (1/500, Sigma). Signals were detected by chemiluminescent reaction with Immobilon Western reagent (Millipore) and a Fusion FX7 (Vilber-Lourmat, France). The intensity of KCNN4 bands relative to the β1 Na,K-ATPase signal was quantified using ImageJ Version 1.44 software (NCBI).

**HEK293 cells transfection:** HEK293 cells were grown in DMEM glutamax (Gibco) 10%FBS penicillin-streptomycin. Cells were co-transfected with 1 μg of WT or point mutated pcDNA3-KCNN4-HA and 0.5 μg of pIRES-eYFP using CaPO4.16 hours later, cells were washed twice with PBS and patch-clamp recordings on fluorescently labeled cells.

**Patch-clamp electrophysiology:** Glass pipettes (Brand, Wertheim, Germany) were made on a horizontal pipette puller (P-97; Sutter Instrument Co.; Navato, CA) to give a final resistance ranging from 3 to 5 MΩ. For whole cell experiments the bath solution was in mM: NaCl 140, KCl 5, $CaCl_2$ 1, Glucose 29, Hepes 25 pH 7.4 adjusted with NaOH. The intracellular solution was in mM: KCl 30, KGluconate 100, EGTA 5, Hepes 10 pH 7.2 adjusted with NaOH, $CaCl_2$ 4.19 (corresponding to 1μM free calcium), MgATP 2. Currents were measured at room temperature using a ramp protocol form -120 to +80 mv from a holding potential of -60mV (sampling frequency 10 kHz; filtered 1 kHz)

**Inside-out recordings:** Calcium-dependence of KCNN4 was studied with intracellular (bath) solutions in mM: KCl 30, KGluconate 100, EGTA 5, Hepes 10 pH 7.2 adjusted with KOH, $CaCl_2$ with varying concentrations 4.91; 4.19; 3.61; 2.82; 1.7 ( 10-5; 10-6; 5.10-7; 2.5.10-7; 10-7 M of free calcium). Maxchelator was used to calculate free $Ca^{2+}$ concentration (http://maxchelator.stanford.edu/CaEGTA-TS.htm). Extracellular solution in mM: NaCl 140, KCl 5, $CaCl_2$ 1, Glucose 29, Hepes 25 pH 7.4 adjusted with NaOH. Currents were evoked by voltage ramps from -120 to 80 mV (150 ms), filtered at 1 kHz and acquired with a sampling frequency of 10 kHz. All traces were corrected for liquid junction potential. For dose response experiments, normalized values of currents at -45 mV were plotted against free $Ca^{2+}$ concentration.

All patch-clamp experiments were performed with a PC-controlled EPC 9 patch-clamp amplifier (HEKA, Lambrecht/Pfalz, Germany). Currents were acquired and analyzed with Pulse and Pulsefit softwares (HEKA).

**Immunohistochemistry:** Immunodetection of KCNN4-HA in HEK293 cells was performed using anti-HA antibody (Sigma-Aldrich).

**Red cell cation content and volume measurements:** Fresh venous blood was obtained by venipuncture from an informed patient from family 1 and a healthy volunteer. For 24 hours' incubation, blood samples were stored at 37°C or 4°C.

For vanadate experiments: blood was washed 4 times at room temperature in medium containing (in mM): NaCl (147) KCl (5) MgSO4 (2) $CaCl_2$ (1) Hepes/NaOH pH7.4 (10). Red cell suspension was then incubated at 37°C, 30% hematocrit and 5 mM vanadate was added alone or with 10 μM TRAM-34. A few minutes before sampling time, 400 μl of cell suspension were taken to fill 3 nylon tubes that were centrifuged for 10 minutes at 4°C, 20000 g at the exact sampling time. The supernatant was collected for extracellular ion content measurements. The pellet of red cells was extracted and immediately weighted wet. Dry weight was measured after overnight heating (80°C). Water content was calculated with a correction of 3.64% corresponding to trapped medium between packed cells. Intracellular ions were extracted from dried pellets by overnight incubation at 4°C in 5 ml milliRho water (Millipore). $Na^+$ and $K^+$ were measured by flame spectroscopy with an Eppendorf ELEX6361.

## 2. Results

It was initially investigated a fetus (proband 1) for severe *in utero* anemia without edema, requiring 1 transfusion *in utero* at week 27 (Hb: 30 g/l). After preterm birth, he received 3 additional transfusions: immediately after birth, at 2 weeks (Hb: 65 g/l) and at 6 weeks of age (Hb: 70 g/l) and was then treated with EPO for 6 weeks. Under treatment, the reticulocyte count progressively increased and Hb value stabilized at 90g/l at 3 months of age. No further transfusion was necessary. Currently, at 4 years 10 months of age, the proband demonstrated mild anemia and splenomegaly. Clinical history revealed that the mother's proband was affected with a chronic moderate hemolytic anemia of unknown origin from childhood. She was treated with regular transfusion regimen from infancy to adolescence. Chelation therapy was started at 8 years of age and a splenectomy performed at 25 years old. During adult life, she received 2 transfusions, one after a delivery and another one during an infection by the parvovirus B19. Four other members of this family (Family 1) originated from France, were also affected by chronic hemolytic anemia (Figure 1A). Three out of 4 were splenectomized and 2 of them have received regular transfusions and chelation therapy in periods of time.

In a second unrelated family (Family 2) the proband (proband 2), a 25 year-old person, has suffered from moderate chronic hemolytic anemia since early childhood. She was never transfused and underwent a cholecystectomy because of biliary lithiasis. Her father was originated from Poland and was reported to have severe hemolytic anemia treated by splenectomy and occasional transfusions. Her 2 year-old son was born after a normal pregnancy carried to term, he also presented with a well-tolerated chronic hemolytic anemia. The hematological parameters of proband 1, his mother, proband 2 and her son are summarized in table 1 below. In addition to anemia, all 4 have a discrete increase of MCHC value.

Table 1: Hematological parameters for 4 subjects carrying the KCNN4 c.1055G>A mutation (representative values in steady state conditions) values in steady state conditions)

| Normal range for adults | Hb (g/l) 130-160 | MCV (fl) 80-100 | Reticulocytes count (G/l) 20-80 | MCHC(g/l) 310-350 | Platelets (G/l) 150-400 | Ferritin (μg/l) 22-322 |
|---|---|---|---|---|---|---|
| Proband 1 (age 4) | 98 | 87.9 | 263 | 356 | 319 | 116 |
| Proband 1 mother | 85 | 109 | 255 | 354 | 783 | 94 |
| Proband 2 | 110 | 93.1 | 249 | 361 | 230 | Nd |
| Proband 2's son (age 2) | 104 | 86.9 | 363 | 365 | 464 | 121 |

A microscopic examination of blood smears from proband 1 showed mild anisopoikilocytosis with less than 1% of target cells, polychromatophilic red blood cells, teardrop cells, elliptocytes with sometimes abnormal hemoglobin distribution, hemighosts, bite cells, knizocytes, schizocytes and rare stomatocytic red cells (Figure 1B). For his mother and for proband 2, anomalies were similar with more significant anisopoikilocytosis and the presence of acanthocytes (Figure 1B). There was no basophilic stippling of red blood cells.

The EMA test, electrophoresis of red cell membrane proteins and hemoglobin study were normal for all of them. The diagnosis of xerocytosis was not retained initially as there was almost no stomatocyte on blood films and repeated ektacytometry was considered as normal for all 4 tested affected individuals.

Whole exome sequencing was performed for 3 subjects in Family 1, the proband, his affected mother and his unaffected sister. Variants were filtered against dbSNP137 and for heterozygous exonic mutations present in affected individuals only. Thirty-three genes were found carrying exonic, non-synonymous heterozygous mutations among which KCNN4 encoding the Gardos channel, was the most consistent candidate because of its expression in red cells. The missense mutation c.1055G>A (p.Arg352His), confirmed by Sanger sequencing, is located in the Calmodulin interacting region and involves a residue highly conserved among species (Figure 1C); it was predicted pathogenic by in silico analysis (Polyphen: http://genetics.bwh.harvard.edu/pph2), with a score of 0.992 for a maximum of 1. Mutation segregation was studied in 3 other members of Family 1, two affected and one unaffected by chronic hemolysis and was consistent with a dominant transmission of the phenotype linked to the mutation. After direct sequencing of KCNN4 in the 2 affected subjects of Family 2, the same missense mutation c.1055G>A, was identified in heterozygous condition for both of them. Splicing was not affected by the substitution as normal-sized transcripts were heterozygotes for the mutation (Figure 1D). Using the data of exome sequencing in proband 1, it was confirmed that no mutation was present in FAM38, encoding PIEZO1 and described as the major cause of HX up to now.

Further investigations were then performed for proband 1 and his mother. Osmotic fragility was tested to check red cells dehydration. Both mother and son had an abnormal profile after 24 hours of incubation at 37°C: 50% red cells lysis was obtained with reduced salt concentration when compared to a normal control (Figure 1E). The profiles were similar to normal control when the same analysis is performed after 24h at 4°C explaining why ektacytometry was normal as it was performed after incubation at 4°C. Plasmatic $K^+$ concentrations in various conditions of time and temperature after sampling were measured by potentiometry and were in normal ranges. Dynamic efflux of $K^+$ under $Ca^{2+}$ stimulation was assessed by 39K NMR of erythrocytes suspensions using stimulation by the ionophore A23187. Except for a short delay in $K^+$ exit following $Ca^{2+}$ activation, no perturbation was observed (data not shown).

In addition, dehydrated red cells are usually associated with haemolytic anemia because shrinkage stimulates Phosphatidylserine (PS) exposure as previously shown in both normal red blood cells, G6PD deficient cells and HbS cells (Lang *et al.*, 2004; Weiss *et al.*, 2011). A relationship between cation leakage and hemolytic anemia has also been observed for other membrane proteins mutations including Band 3 mutations (Bruce *et al.*, 2005). In the present study, it was observed in the patients with mutation in the Gardos channel, a variability in disease severity with a level of anemia rather severe in Family 1 whereas individuals of Family 2 and individuals with PIEZO1 mutations, present normal or subnormal Hb levels (Carella *et al.*, 1998; Houston *et al.*, 2011). Indeed, 2 of the affected individuals from Family 1 exhibit pronounced anemia with an extremely severe episode of *in utero* anemia for the proband (with no other identified cause, especially no maternal-fetal incompatibility) and numerous transfusions required at many occasions, for his mother. This suggests that susceptibility to scramblase activation resulting from prolonged $Ca^{2+}$ activation and leading to PS exposure may be enhanced in these patients. Iron overload due to chronic anemia is difficult to evaluate in Family 1 as the mother's proband has regularly been transfused and treated by Deferriprox and the proband himself is too young to suffer from iron overload. In the second family, the patients exhibit moderate iron overload as observed in chronic hemolytic anemia.

The function of the Gardos channel variant p.Arg352His was then investigated by the expression of a plasmid containing the mutated cDNA in *Xenopus laevis* oocytes. The current voltage curves showed that the missense mutation p.Arg352His does not prevent ion conductance through the channel when compared to the wild type channel (Figure 2A). The activation phase of WT and p.Arg352His channel induced by calcium ionophore was similar but, whereas WT KCNN4 activity decreases after reaching a maximum, the p.Arg352His mutant activity remains quite constant for several minutes. The high and sustained currents with p.Arg352His made it difficult to record for more than 2 minutes after the peak. The reversion potential (-120 mV) was similar between WT and the mutated channel but the current elicited by p.Arg352His KCNN4 expression in *Xenopus* oocyte was higher than observed with WT (Figure 2B). Western blots confirmed that both proteins are expressed at similar levels, suggesting that the conductance increase observed for the mutated channel is directly associated with the mutation. These data indicate that the mutation alters the regulation of channel activity favoring a longer activated-state. Inhibition tests performed with TRAM-34, the classical inhibitor of KCNN4, result in decreased current production indicating that the p.Arg352His variant is sensitive to inhibition (Figure 2C).

To further characterize the p.Arg352His KCNN4, HEK293 cells were transfected with WT or mutated channel. The mutation does not prevent addressing of the channel to plasma membrane. Whole-cell recording shows a different calcium dependent-activation kinetic for HEK293 cells expressing WT or p.Arg352His KCNN4 (Figure 3A). For the former, the current progressively appears while $Ca^{2+}$ diffuses from the pipette to the intracellular compartment. By contrast, in the latter case, the current is activated immediately after break-in for the mutant and further increases during the time of recording. As in oocyte experiments, the maximum current density is increased in p.Arg352His KCNN4 expressing cells (Figure 3A-C). These results suggest that the mutation increases channel sensitivity to calcium. The leftward shift in reversal potential observed between break-in and steady-state for WT confirms the delay due to $Ca^{2+}$ diffusion to activate the channel. This delay is not observed for p.Arg352His KCNN4. The calcium sensitivity of WT versus mutated KCNN4 was further explored by performing giant excised inside-out patch-clamp experiments. Figure 3D shows representative traces of $K^+$ currents as a function of voltage and $Ca^{2+}$ concentrations applied to the internal face of the membrane. In Figure 3E, currents at -45 mV are plotted as a function of $Ca^{2+}$ concentrations. Quantitative analysis showed the calcium dependence of the WT KCNN4 to have an apparent Kd of 0.95 $\mu$M $\pm$ 0.09 whereas the apparent Kd is 0.21 $\mu$M $\pm$ 0.02 for p.Arg352His mutant. The Hill coefficients are not statistically different between WT and mutant KCNN4 (3.75 $\pm$ 1.45 and 3.3 $\pm$ 0.85 respectively, n=4).

According to electrophysiological data, KCNN4 should be activated by lower calcium concentration in patient red cells compared to control. To assess the effect of an increase in intracellular $Ca^{2+}$ on the kinetic of Gardos channel activation in control or patient red cells, the net potassium flux was measured in red cells treated by vanadate. Vanadate increases intracellular $Ca^{2+}$ concentration in red cells by inhibiting the calcium pump and also by activating the calcium influx (Varecka and Carafoli, 1982; Bennekou *et al.*, 2012). Figure 4 illustrates the $K^+$ content of control or patient red cells in presence of 5mM vanadate with or without 10 $\mu$M TRAM-34. Whereas vanadate did not significantly change intracellular $K^+$ content in control red cells in 1 hour, a significant decrease in intracellular $K^+$ was observed in patient red cells and this decrease was blocked by TRAM-34. The $K^+$ efflux is correlated to cell volume decrease as illustrated on Figure 4B. No significant change in $Na^+$ contents was observed in control and in patient red cells at the same time (Figure 4C).

The $K^+$ content of red cells in blood stored for 24 hours at 37°C or 4°C is given in table 2 below.

Table 2: $K^+$ content in red cells as a function of blood temperature. Data are expressed in $\mu$mol per gram of dry weight ($\mu$mol/g d.w. +/- S.D. for 3 samples).

| $K^+$ content | t0 | 24 h 37°C | 24 h 4°C |
|---|---|---|---|
| Control | 281.1 +/-4.9 | 260.0 +/-1.7 | 257.0 +/-3.4 |
| Proband l'aunt (affected) | 316.9 +/- 5.5 | 223.1 +/-10.2 | 281.6 +/- 4.6 |

In control red cells, the $K^+$ content is decreased by 21.1 $\mu$mol/g d.w. after 24h at 37°C. This variation is similar for blood stored for 24h at 4°C (-24.1 $\mu$mol/g d.w.). In contrast, there is a $K^+$ loss of 93.8 $\mu$mol/g d.w. in patient red cells stored at 37°C compared to 35.3 $\mu$mol/g d.w. for patient blood stored for 24h at 4°C.

**REFERENCES**

Andolfo I, et al., 2013a, Blood. 121:3925-3935.
Andolfo I, et al., 2013b, Am J Hematol. 88:66-72.
Archer NM, et al., 2014, Am J Hematol. 89:1142-1146.
AtagaKI, et al., 2008, Blood. 111:3991-3997.
Ataga KI, et al., 2009, Expert Opin Investig Drugs. 18:231-239.
Ataga KI, et al., 2011, Br J Haematol. 153:92-104.
Bae C, et al., 2013, Proc Natl Acad Sci U S A. 110:E1162-1168.
Barneaud-Rocca et al., 2011, J Biol. Chem. 286:8909-8916.
Bennekou P, et al., 2012. Blood Cells Mol. Dis. 48:102-109.
Bruce LJ, et al., 2005, Nat Genet. 37:1258-1263.
Brugnara C, et al., 1993a, J Clin Invest. 92:520-526.
Brugnara C, et al., 1993b, J. Biol. Chem. 268:8760-8768
Brugnara C, et al., 1996, J Clin Invest. 97:1227-1234.
Carella M, et al., 1998, Am J Hum Genet. 63:810-816.
Castle A, et al., 2002, Mol. Pharmacol. 63:409-18.
Da Costa L, et al., 2013, Blood Rev. 27:167-178.
Dyrda A, et al., 2010, PLoS One. 5:e9447.
Ellory JC, et al., 1994, Br J Pharmacol. 111:903-905
Fanger CM, et al., 1999, J Biol Chem. 274:5746-5754.
Houston BL, et al., 2011, Blood Cells Mol Dis. 47:226-231.
Joiner WJ, et al., 1997, Proc Natl Acad Sci U S A. 94:11013-11018.
Joiner WJ, et al., 2001, J Biol Chem. 276:37980-5.
Kaplan JM and Delpech M., 2007, Biologie moleculaire et medecine (3° Éd.) (Coll. De la biologie à la clinique), Ed. Flammarion
Lang F, et al., 2004, Adv Exp Med Biol. 559:211-217.
Maher AD, Kuchel PW, 2003, Int J Biochem Cell Biol. 35:1182-1197.
Martial S, et al., 2007, J Cell Physiol. 213:70-78.
Miller DR, et al., 1971, Blood. 38:184-204.
Morales P, et al., 2013, J Gen Physiol. 142:37-60.
Quinlan AR and Hall IM, 2010, Bioinformatics. 26:841-842.
Rinehart J, et al., 2010, Curr Opin Hematol. 17:191-197.
Romero JR, et al., 2002, Blood. 99:1103-1108.
Stocker W, et al., 2003, Blood. 101:2412-2418.
Varecka L and Carafoli E., 1982, J Biol Chem. 257:7414-7421.
Wang K, et al., 2010, Nucleic Acids Res.;38: e164.
Weiss E, et al., 2011, Anemia. 2011:379894.
Wulff H, Castle NA, 2010, Expert Rev Clin Pharmacol. 3:385-396.
WulffH, et al., 2000, PNAS. 97:8151-8156.
Wulff H, Köhler R, 2013, J Cardiovasc Pharmacol. 61:102-112.
Zarychanski R, et al., 2012, Blood. 120:1908-1915.

## Claims

1. An inhibitor of the Gardos channel (KCNN4 protein) for use in the treatment of hereditary xerocytosis, wherein said inhibitor is a selective Gardos channel blocker that specifically inhibits the efflux of potassium from the erythrocytes.

2. The inhibitor for use according to claim 1, **characterized in that** it is selected from the group consisting of an organic molecule, an amino acid and an antibody.

3. The inhibitor for use according to claim 2, **characterized in that** it is selected from the group consisting of imidazole antimycotics, clotrimazole, metronidazole, econazole, arginine, Tram-34, harybdotoxin, nifedipine, 2,2-Bis(4-fluorophenyl)-N-methoxy-2-phenylacetamidine, 2-(2-Chlorophenyl)-2,2-diphenylacetaldehyde oxime, 2-(2-Chlorophenyl)-2,2-bis(4-fluorophenyl)-N-hydroxyacetamidine, 2,2,2-Tris(4-fluorophenyl)-N-hydroxyacetamidine, 2-(2-Fluorophenyl)-2-(4-fluorophenyl)-N-hydroxy-2-phenylacetamidine, phosphoric acid 3-(2-oxazolyl)-4-[3-(trifluoromethyl)phenylsulfonamido]phenyl monoester, N-[2-(4,5-Dihydrooxazol-2-yl)phenyl]-3-(trifluoromethyl)benzenesulfonamide, N-[4-Methoxy-2-(2-oxazolyl)phenyl]benzenesulfonamide, N-[4,5-Dimethoxy-2-(3-methyl-1,2,4-oxadiazol-5-yl)phenyl]-3-(trifluoromethyl)benzenesulfonamide, N-[2-(2-Furyl)phenyl]-3-(trifluoromethyl)benzenesulfonamide and N-[4-Methyl-2-(2-oxazolyl)phenyl]-3μ-(trifluoromethyl)benzenesulfonamide and senicapoc.

4. The inhibitor for use according to any one of claims 1 to 3, **characterized in that** it is used in the treatment of hereditary xerocytosis of a human subject who is a carrier of the missense mutation c.1055G>A in the *KCNN4* gene encoding the Gardos channel, resulting in an amino acid change from arginine to histidine in codon 352.

5. A method for genotyping, *in vitro,* the *KCNN4* gene in a human subject comprising the steps of:

   (a) isolating mRNA or genomic DNA from a nucleic acid sample obtained from said subject,
   (b) determining the nucleotide present at position c.1055 of the *KCNN4* gene encoding the Gardos channel.

6. The method according to claim 5, **characterized in that** the mRNA or genomic DNA is obtained from a blood sample from said subject.

7. The method according to claim 5 or claim 6, **characterized in that** the mRNA is obtained from reticulocytes from said subject and the genomic DNA is obtained from white blood cells from said subject.

8. The method according to any one of claims 5 to 7, **characterized in that** said step (b) is carried out by hybridization techniques, selective amplification, nucleic acid sequencing, restriction fragment length polymorphism (RFLP), amplified fragment length polymorphism (AFLP), ligation chain reaction (LCR) or mass spectrometry.

9. The method according to any one of claims 5 to 7, **characterized in that** said nucleotide is determined by reverse dot blot using the probes of SEQ ID NO: 3 and SEQ ID NO: 4.

10. An *in vitro* method of diagnosing the presence of or predisposition to hereditary xerocytosis in a human subject, comprising the step of:

    (i) providing a biological sample from said subject and
    (ii) detecting the presence of the missense mutation c.1055G>A in the *KCNN4* gene encoding the Gardos channel or the missense mutation p.Arg352His in the Gardos channel (KCNN4 protein),

    the presence of said mutation constituting a marker of a hereditary xerocytosis or a predisposition to hereditary xerocytosis in said subject.

11. The method according to claim 10, **characterized in that** the presence of the missense mutation c.1055G>A in the *KCNN4* gene encoding the Gardos channel is detected by genotyping the *KCNN4* gene in said human subject according to the method of any one of claims 5 to 9.

12. The method according to claim 10, **characterized in that** the presence of the p.Arg352His mutation in the Gardos channel is detected by protein sequencing or binding to a ligand specifically directed to the Gardos channel variant p.Arg352His.

**13.** A kit for diagnosing a hereditary xerocytosis comprising the probe of SEQ ID NO: 3 and/or the probe of SEQ ID NO: 4.

**14.** Use of the probe of SEQ ID NO: 3 and/or the probe of SEQ ID NO: 4 *for in vitro* diagnosing a hereditary xerocytosis in a human subject.

**15.** An *in vitro* a method for screening a biologically active inhibitor of the human Gardos channel (KCNN4 protein) variant p.Arg352His, said method comprising contacting *in vitro* a test compound with the human Gardos channel (KCNN4 protein) variant p.Arg352His of SEQ ID NO: 2 and determining the ability of said test compound to prevent ion conductance through the channel when compared to the wild-type human Gardos channel (KCNN4 protein) of SEQ ID NO: 2, wherein preventing ion conductance through the channel when compared to the wild-type human Gardos channel (KCNN4 protein) of SEQ ID NO: 2 provides an indication as to the ability of the compound to inhibit the human Gardos channel (KCNN4 protein) variant p.Arg352His.

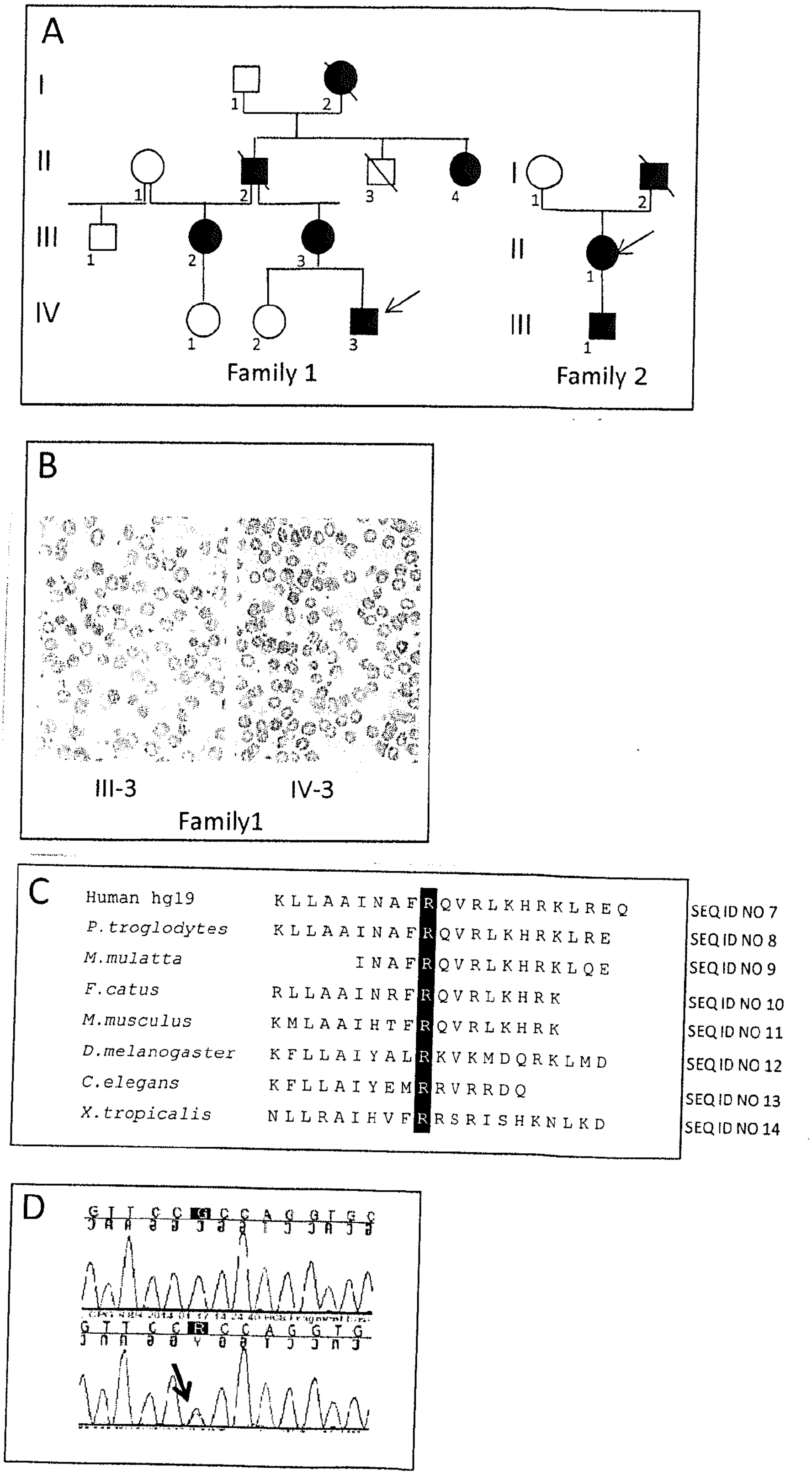
A
I
II
III
IV
Family 1
Family 2
B
III-3
IV-3
Family1
C
Human hg19 KLLAAINAFRQVRLKHRKLREQ SEQ ID NO 7
P.troglodytes KLLAAINAFRQVRLKHRKLRE SEQ ID NO 8
M.mulatta INAFRQVRLKHRKLQE SEQ ID NO 9
F.catus RLLAAINRFRQVRLKHRK SEQ ID NO 10
M.musculus KMLAAIHTFRQVRLKHRK SEQ ID NO 11
D.melanogaster KFLLAIYALRKVKMDQRKLMD SEQ ID NO 12
C.elegans KFLLAIYEMRRVRRDQ SEQ ID NO 13
X.tropicalis NLLRAIHVFRRSRISHKNLKD SEQ ID NO 14
D

Figure 1

**E**

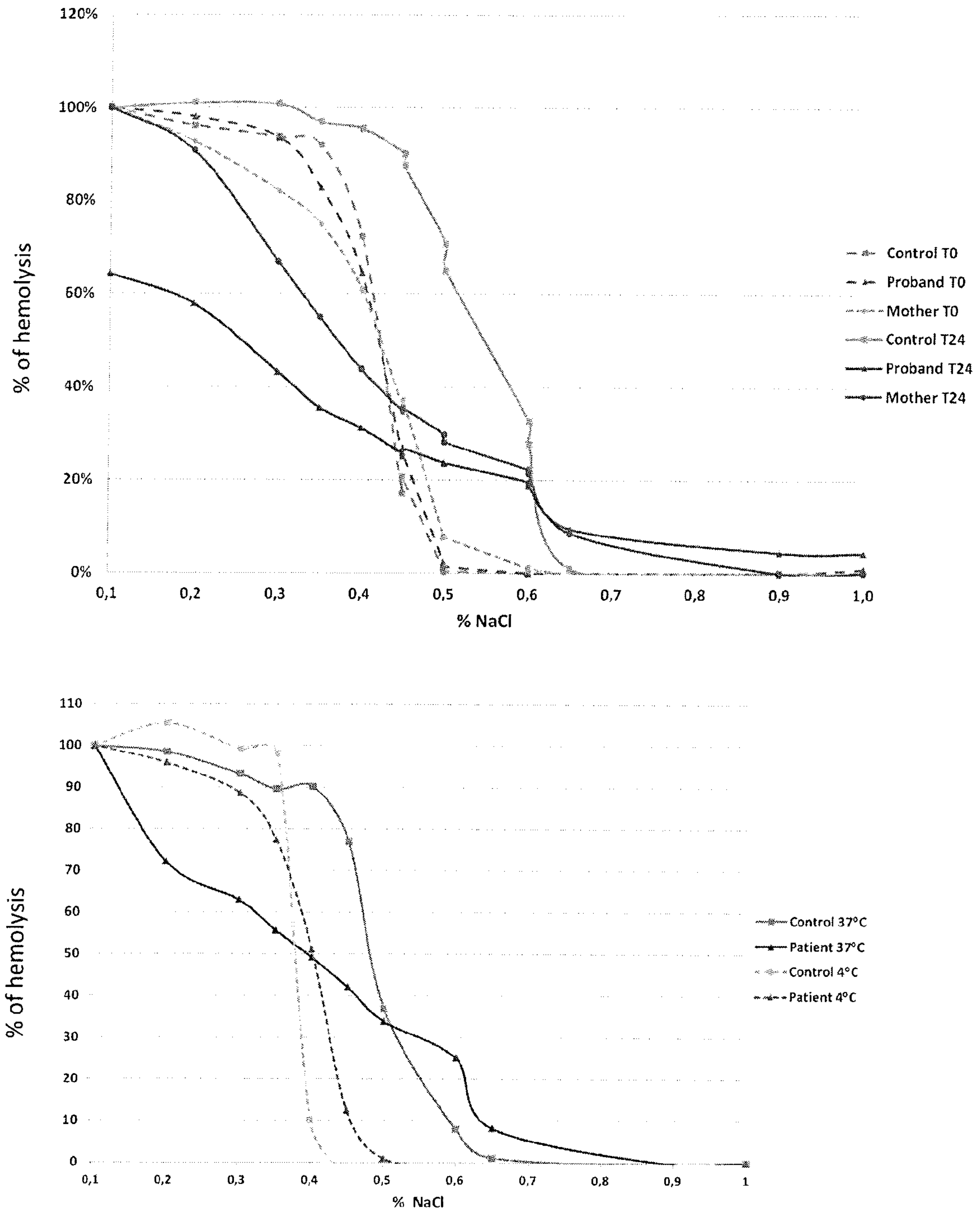


**Figure 1 (suite)**

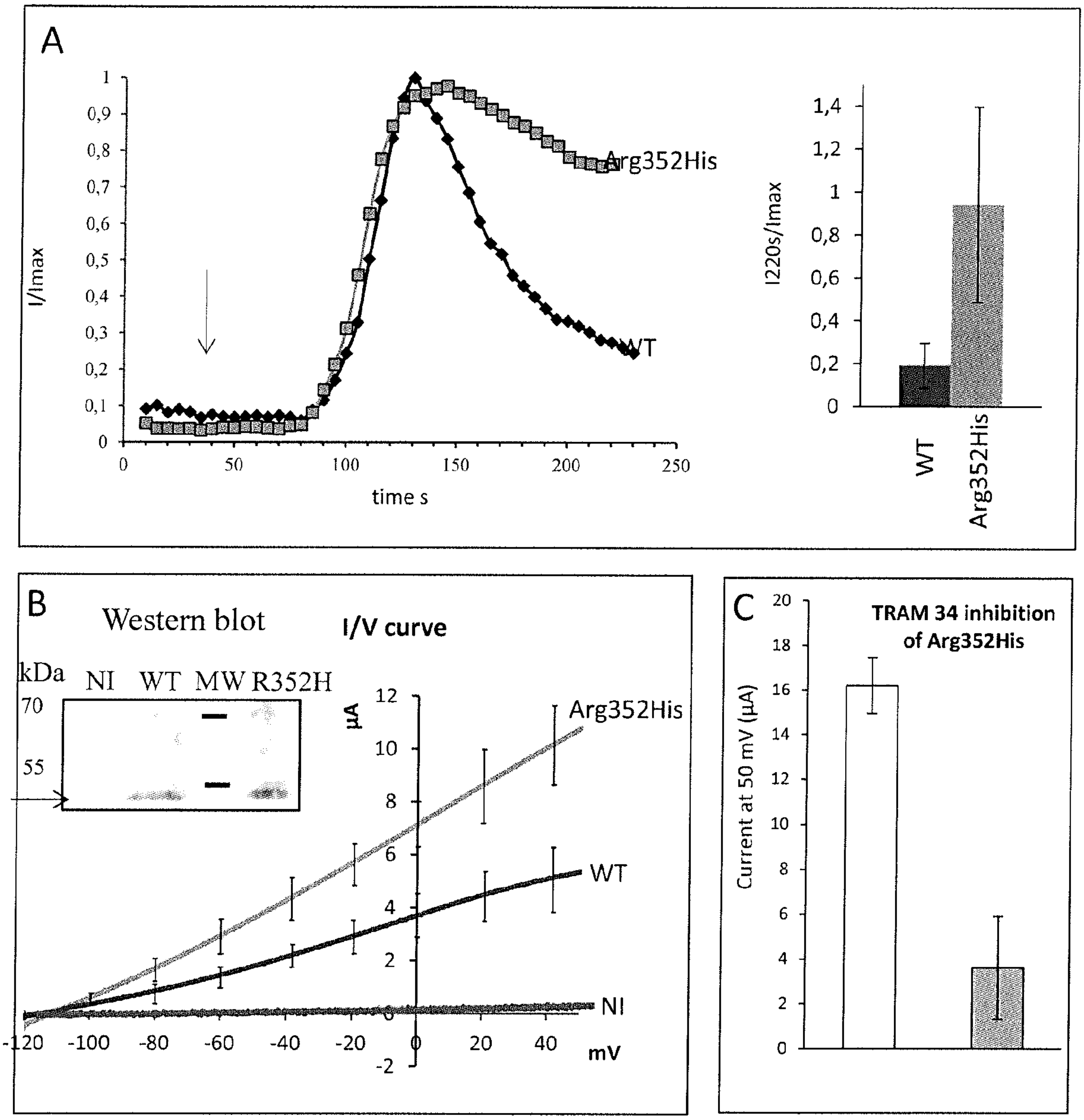
A
I/Imax
time s
Arg352His
WT
I220s/Imax
WT
Arg352His
B
Western blot
kDa
NI WT MW R352H
70
55
I/V curve
µA
Arg352His
WT
NI
mV
C
TRAM 34 inhibition of Arg352His
Current at 50 mV (µA)

**Figure 2**

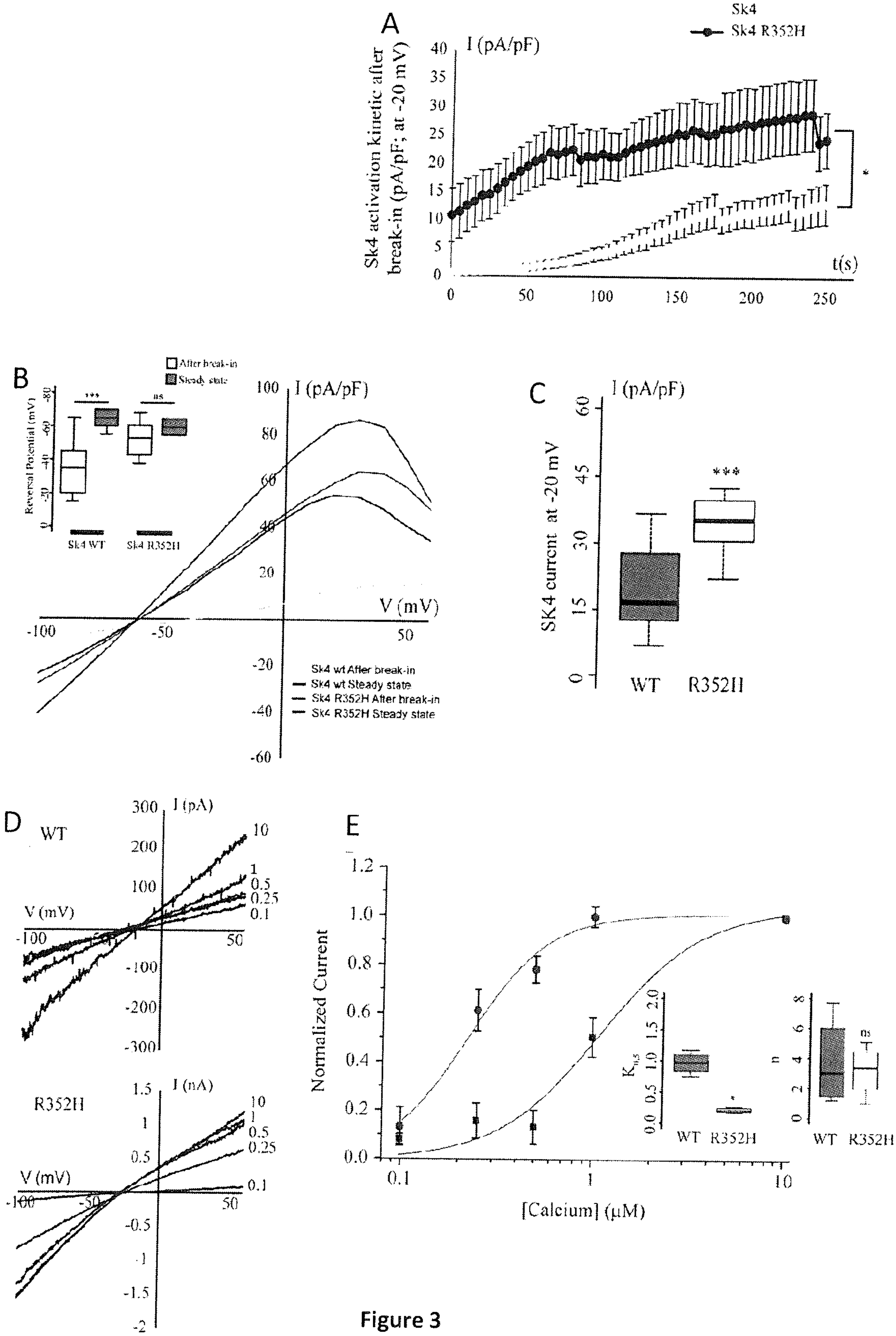
A
Sk4
Sk4 R352H
Sk4 activation kinetic after break-in (pA/pF; at -20 mV)
I (pA/pF)
t(s)
B
After break-in
Steady state
Reversal Potential (mV)
Sk4 WT
Sk4 R352H
I (pA/pF)
V (mV)
Sk4 wt After break-in
Sk4 wt Steady state
Sk4 R352H After break-in
Sk4 R352H Steady state
C
I (pA/pF)
SK4 current at -20 mV
WT
R352H
D
WT
I (pA)
V (mV)
R352H
I (nA)
E
Normalized Current
[Calcium] (μM)
WT
R352H

Figure 3

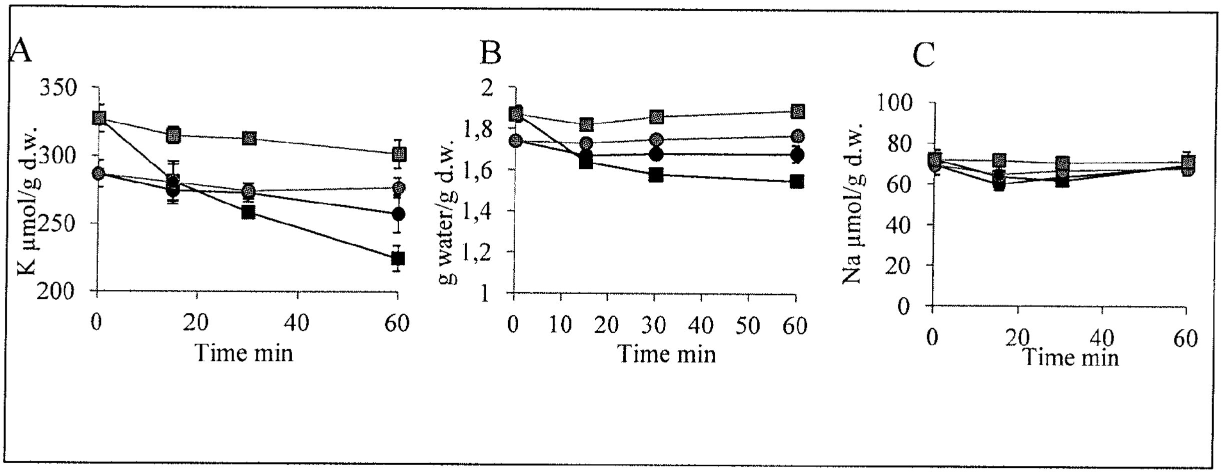
A
350
300
250
200
K µmol/g d.w.
0
20
40
60
Time min
B
2
1,8
1,6
1,4
1,2
1
g water/g d.w.
0
10
20
30
40
50
60
Time min
C
100
80
60
40
20
0
Na µmol/g d.w.
0
20
40
60
Time min

Figure 4

Europäisches Patentamt
European Patent Office
Office européen des brevets

# EUROPEAN SEARCH REPORT

Application Number
EP 15 30 5921

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y<br><br>A | WO 2014/064465 A1 (NHS BLOOD & TRANSPLANT [GB]) 1 May 2014 (2014-05-01)<br>* page 1, lines 5-8 *<br>* claims 1-20 *<br>* page 6, line 14 - page 8, line 2 *<br>----- | 1-4<br><br>5-15 | INV.<br>A61K31/00<br>G01N33/50<br>A61P7/00 |
| Y,D | WO 00/50026 A1 (ICAGEN INC [US]; MCNAUGHTON SMITH GRANT ANDREW [US]; RIGDON GREGORY CO) 31 August 2000 (2000-08-31)<br>* page 3, lines 6-29 *<br>* page 5, lines 12-21 *<br>----- | 1-4 | |
| A | STUART M CAHALAN ET AL: "Piezo1 links mechanical forces to red blood cell volume",<br>ELIFE,<br>vol. 4, 22 May 2015 (2015-05-22), XP055224839,<br>DOI: 10.7554/eLife.07370<br>* abstract *<br>----- | 1-14 | |
| A | US 2008/293623 A1 (DALTON JOAN [GB] ET AL) 27 November 2008 (2008-11-27)<br>* claims 1,7 *<br>----- | 1-14 | TECHNICAL FIELDS SEARCHED (IPC)<br>A61K<br>G01N<br>A61P |

The present search report has been drawn up for all claims

2

| Place of search | Date of completion of the search | Examiner |
|---|---|---|
| Munich | 4 November 2015 | Habedanck, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 15 30 5921

This annex lists the patent family members relating to the patent documents cited in the above-mentioned European search report. The members are as contained in the European Patent Office EDP file on
The European Patent Office is in no way liable for these particulars which are merely given for the purpose of information.

04-11-2015

| Patent document cited in search report | Publication date | Patent family member(s) | Publication date |
|---|---|---|---|
| WO 2014064465 A1 | 01-05-2014 | NONE | |
| WO 0050026 A1 | 31-08-2000 | AP 1465 A | 22-09-2005 |
| | | AT 370728 T | 15-09-2007 |
| | | AU 762039 B2 | 19-06-2003 |
| | | AU 2880700 A | 14-09-2000 |
| | | BR 0008416 A | 29-01-2002 |
| | | CA 2371857 A1 | 31-08-2000 |
| | | CN 1344158 A | 10-04-2002 |
| | | DE 60036093 T2 | 15-05-2008 |
| | | EP 1158971 A1 | 05-12-2001 |
| | | ES 2291191 T3 | 01-03-2008 |
| | | HK 1045262 A1 | 29-07-2005 |
| | | IL 145012 A | 10-12-2006 |
| | | JP 4038337 B2 | 23-01-2008 |
| | | JP 2002537331 A | 05-11-2002 |
| | | JP 2007262075 A | 11-10-2007 |
| | | OA 11838 A | 22-08-2005 |
| | | US 6288122 B1 | 11-09-2001 |
| | | WO 0050026 A1 | 31-08-2000 |
| US 2008293623 A1 | 27-11-2008 | US 2008293623 A1 | 27-11-2008 |
| | | US 2011021418 A1 | 27-01-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0050026 A
- WO 2004016221 A
- WO 2005113490 A
- WO 2006084031 A


### Non-patent literature cited in the description


- **ANDOLFO I et al.** *Blood,* 2013, vol. 121, 3925-3935
- **ANDOLFO I et al.** *Am J Hematol.,* 2013, vol. 88, 66-72
- **ARCHER NM et al.** *Am J Hematol.,* 2014, vol. 89, 1142-1146
- **ATAGAKI et al.** *Blood,* 2008, vol. 111, 3991-3997
- **ATAGA KI et al.** *Expert Opin Investig Drugs,* 2009, vol. 18, 231-239
- **ATAGA KI et al.** *Br J Haematol,* 2011, vol. 153, 92-104
- **BAE C et al.** *Proc Natl Acad Sci U S A.,* 2013, vol. 110, E1162-1168
- **BARNEAUD-ROCCA et al.** *J Biol. Chem.,* 2011, vol. 286, 8909-8916
- **BENNEKOU P et al.** *Blood Cells Mol. Dis.,* 2012, vol. 48, 102-109
- **BRUCE LJ et al.** *Nat Genet.,* 2005, vol. 37, 1258-1263
- **BRUGNARA C et al.** *J Clin Invest.,* 1993, vol. 92, 520-526
- **BRUGNARA C et al.** *J. Biol. Chem.,* 1993, vol. 268, 8760-8768
- **BRUGNARA C et al.** *J Clin Invest.,* 1996, vol. 97, 1227-1234
- **CARELLA M et al.** *Am J Hum Genet.,* 1998, vol. 63, 810-816
- **CASTLE A et al.** *Mol. Pharmacol.,* 2002, vol. 63, 409-18
- **DA COSTA L et al.** *Blood Rev.,* 2013, vol. 27, 167-178
- **DYRDA A et al.** *PLoS One.,* 2010, vol. 5, e9447
- **ELLORY JC et al.** *Br J Pharmacol.,* 1994, vol. 111, 903-905
- **FANGER CM et al.** *J Biol Chem.,* 1999, vol. 274, 5746-5754
- **HOUSTON BL et al.** *Blood Cells Mol Dis.,* 2011, vol. 47, 226-231
- **JOINER WJ et al.** *Proc Natl Acad Sci U S A.,* 1997, vol. 94, 11013-11018
- **JOINER WJ et al.** *J Biol Chem.,* 2001, vol. 276, 37980-5
- **KAPLAN JM ; DELPECH M.** Biologie moleculaire et medecine
- **LANG F et al.** *Adv Exp Med Biol.,* 2004, vol. 559, 211-217
- **MAHER AD ; KUCHEL PW.** *Int J Biochem Cell Biol.,* 2003, vol. 35, 1182-1197
- **MARTIAL S et al.** *J Cell Physiol.,* 2007, vol. 213, 70-78
- **MILLER DR et al.** *Blood,* 1971, vol. 38, 184-204
- **MORALES P et al.** *J Gen Physiol.,* 2013, vol. 142, 37-60
- **QUINLAN AR ; HALL IM.** *Bioinformatics,* 2010, vol. 26, 841-842
- **RINEHART J et al.** *Curr Opin Hematol.,* 2010, vol. 17, 191-197
- **ROMERO JR et al.** *Blood,* 2002, vol. 99, 1103-1108
- **STOCKER W et al.** *Blood,* 2003, vol. 101, 2412-2418
- **VARECKA L ; CARAFOLI E.** *J Biol Chem.,* 1982, vol. 257, 7414-7421
- **WANG K et al.** *Nucleic Acids Res.,* 2010, vol. 38, e164
- **WEISS E et al.** *Anemia,* 2011, vol. 2011, 379894
- **WULFF H ; CASTLE NA.** *Expert Rev Clin Pharmacol.,* 2010, vol. 3, 385-396
- **WULFFH et al.** *PNAS,* 2000, vol. 97, 8151-8156
- **WULFF H ; KÖHLER R.** *J Cardiovasc Pharmacol.,* 2013, vol. 61, 102-112
- **ZARYCHANSKI R et al.** *Blood,* 2012, vol. 120, 1908-1915